# EUROPEAN PATENT APPLICATION

(11) **EP 2 907 486 A1**
(43) Date of publication of application: **19.08.2015**
(21) Application number: 15160040.0
(22) Date of filing: 10.02.2011
(51) Int. Cl.: A61F 5/00

(54) **REMOTELY ADJUSTABLE GASTRIC BANDING SYSTEM**

(30) Priority: 12.02.2010 US 705245
(62) Divisional of application: 11704161.6
(71) Applicant: APOLLO ENDOSURGERY, INC., Austin, TX 78746 (US)
(72) Inventor: Birk, Janel, Oxnard, CA California 93036 (US); Leslie, Dustin B., Santa Barbara, CA California 93110 (US); Snow, Sean, Carpinteria, CA California 93013 (US); Tezel, Ahmet, Goleta, CA California 93117 (US)
(74) Representative: Hoffmann Eitle

(57) **Abstract**

The present invention relates to an implantable, motorized device that facilitates movement of fluid to an inflatable portion of a gastric band, the implantable, motorized device comprising: a housing having a fluid port; an integrated reservoir positioned within the housing and adjacent to the fluid port and capable of holding the fluid; a first piston positioned within the housing and adjacent to the integrated reservoir, the first piston capable of moving a portion of the fluid from the integrated reservoir to the inflatable portion of the gastric band; a first leadscrew coupled to the first piston and causing the first piston to move between a first position and a second position; and a motor attached to the housing and coupled to the first leadscrew, the motor moving the first piston between the first position and the second position, and when the first piston is in the first position, the integrated reservoir having a larger volume than when the first piston is in the second position, the motor being controlled by a telemetric signal received from a remote device.

## Description

### RELATED APPLICATION

This application claims the benefit of U.S. Patent Application Serial Number 12/705,245, filed on February 12, 2010, the entire disclosure of which is incorporated herein by this specific reference.

### FIELD

The present invention generally relates to medical systems and apparatus and uses thereof for treating obesity and/or obesity-related diseases, and more specifically, relates to gastric banding systems that are remotely adjustable.

### BACKGROUND

Adjustable gastric banding apparatus have provided an effective and substantially less invasive alternative to gastric bypass surgery and other conventional surgical weight loss procedures. Despite the positive outcomes of invasive weight loss procedures, such as gastric bypass surgery, it has been recognized that sustained weight loss can be achieved through a laparoscopically-placed gastric band, for example, the LAP-BAND® (Allergan, Inc., Irvine, CA) gastric band or the LAP-BAND AP® (Allergan, Inc., Irvine, CA) gastric band. Generally, gastric bands are placed about the cardia, or upper portion, of a patient's stomach forming a stoma that restricts food's passage into a lower portion of the stomach. When the stoma is of an appropriate size that is restricted by a gastric band, food held in the upper portion of the stomach provides a feeling of satiety or fullness that discourages overeating. Unlike gastric bypass procedures, gastric band apparatus are reversible and require no permanent modification to the gastrointestinal tract.

Over time, a stoma created by a gastric band may need adjustment in order to maintain an appropriate size, which is neither too restrictive nor too passive. Accordingly, prior art gastric band systems provide a subcutaneous fluid access port connected to an expandable or inflatable portion of the gastric band. By adding fluid to or removing fluid from the inflatable portion by means of a hypodermic needle inserted into the access port, the effective size of the gastric band can be adjusted to provide a tighter or looser constriction. Naturally, it would be desirable to allow for non-invasive adjustment of gastric band constriction, for example, without the use of a hypodermic needle.

Birk, et al., U.S. Patent Pub. No. 2010-0010291, which is commonly-assigned and co-pending with the present application, is incorporated herein in its entirety by this specific reference. Birk discloses certain approaches to implantable pumping systems that may be relevant.

Some attempts have been made to cause weight loss by deploying an inflatable bladder within the stomach. For example, Brown, U.S. Patent No. 5,259,339 discloses a bladder that may be inflated and deflated within the stomach to control appetite. Brown utilizes a pump that is external to the patient's body. The external pump is connected to the bladder via a percutaneous endoscopic gastronomy tube which creates a permanent channel to the stomach. Brown does not disclose use of the tube in connection with a gastric band.

Some attempts to develop implantable pump systems have used electromagnetic motors to drive the pump systems. For example, Larson, Jr., et al., U.S. Patent No. 5,676,162, discloses a permanent magnet linear electric motor to assist the heart. Such electromagnetic motors can have the drawback of using strong permanent magnets which may affect magnetic resonance (MRI) procedures. Additionally, such motors may lack a gearing system that keeps the piston seal stationary during periods when the electrical source is removed.

Franetzki, et al., U.S. Patent No. 4,883,467, discloses a reciprocating pump for use in an implantable medication dosage device capable of delivering small boluses of drugs that have a volume less than ten micro-liters. Franetzki discloses a device that utilizes an electromagnetic system comprising strong permanent magnets and a high current electromagnet. Also, Franetzki discloses one-way movement of the medication into the patient.

Additionally, Goldowsky, U.S. Patent No. 5,360,445, discloses an implantable blood pump actuator for an artificial heart. The actuator utilizes a voice coil linear electromagnetic motor to power a hydraulic piston.

Some existing pump systems utilize bendable components that are subject to fatigue and failure. For example, Hassler, Jr., et al., U.S. Patent No. 7,374,565 discloses an implantable artificial sphincter system that utilizes a transcutaneous energy transfer for adjustment of the sphincter. A propellant causes a metal bellows to expand or collapse, and the opposite movement is effected by a thermal element that heats or cools the propellant. The repeated expanding and collapsing of the bellows may subject it to fatigue and failure.

Thus, there continues to remain a need for more effective implantable pump systems for use with adjustable gastric bands, particularly such implantable pump systems with increased accuracy in pressure/flow measurements and with increased and more efficient pumping capability.

### SUMMARY

Generally described herein are remotely adjustable and powered gastric banding systems, and methods of use thereof. The apparatus, systems and methods described herein aid in facilitating obesity control and/or treating obesity-related diseases while being non-invasive once implanted.

In one embodiment, an implantable device is configured for filling and draining an inflatable portion of a gastric band. The implantable device comprises a reservoir for holding a fluid and a sensor positioned between the gastric band and the reservoir. The sensor has a first inlet and a second inlet, and the fluid flows from the first inlet to the second inlet when filling the inflatable portion of the gastric band. The fluid flows from the second inlet to the first inlet when draining the inflatable portion of the gastric band. The sensor monitors a parameter of the fluid when filling and draining the inflatable portion of the gastric band.

The implantable device further comprises a first flow control device that is coupled to the first inlet of the sensor. The first flow control device controls a flow of the fluid when filling and draining the inflatable portion of the gastric band. Additionally, the implantable device comprises a pumping device coupled between the reservoir and the first flow control device. The pumping device moves the fluid into and out of the inflatable portion of the gastric band, and the pumping device is a non-magnetic electrical drive capable of being activated and deactivated using a telemetric signal received from a remote device.

In accordance with another embodiment, an implantable, motorized piston pump comprises a housing with a fluid port and an integrated reservoir capable of holding a fluid. The reservoir is positioned within the housing and adjacent to the fluid port. The implantable pump further comprises a first piston positioned within the housing and adjacent to the integrated reservoir. The first piston is capable of moving a portion of the fluid from the integrated reservoir to the inflatable portion of the gastric band.

Additionally, the implantable pump comprises a first leadscrew coupled to the first piston, and the first leadscrew is capable of causing the first piston to move between a first position and a second position within the housing. A motor is attached to the housing and coupled to the first leadscrew, and the motor moves the first piston between the first position and the second position. When the first piston is in the first position, the integrated reservoir has a larger volume than when the first piston is in the second position. In an embodiment, the motor is capable of being controlled by a telemetric signal received from a remote device.

In accordance with an embodiment, the implantable pump may comprise a slidable seal connected to the first piston. The slidable seal maintains a seal with the housing as the first piston moves from the first position to the second position.

According to yet another embodiment, an implantable, motorized device comprises a housing with a first fluid port, a second fluid port, and a first reservoir positioned within the housing and adjacent to the first fluid port. A second reservoir is also positioned within the housing and is positioned adjacent to the second fluid port. The first reservoir and the second reservoir are both capable of holding a portion of the fluid.

A first piston is positioned within the housing and adjacent to the first reservoir, and the first piston is capable of moving a portion of the fluid out of the first reservoir into the inflatable portion of the gastric band. A second piston is positioned within the housing and is positioned adjacent to the second reservoir. The second piston is capable of moving a portion of the fluid into the second reservoir.

The implantable device further comprises a bellows disposed between the first piston and the second piston. A motor is located within the bellows and is coupled to the first piston and the second piston. The motor facilitates moving the first piston and the second piston within the housing, and the bellows is hermetically sealed and expands and contracts as the first piston and the second piston move within the housing.

### BRIEF DESCRIPTION OF THE DRAWINGS

FIG. 1 illustrates a gastric banding system according to an embodiment of the present invention.
FIG. 2 illustrates a gastric banding system according to another embodiment of the present invention.
FIG. 3 illustrates a gastric banding system according to yet another embodiment of the present invention.
FIG. 4 illustrates a cross-sectional view of an implantable pump according to an embodiment of the present invention.
FIG. 5 illustrates a schematic representation of an implantable pump with a flexible bladder according to an embodiment of the present invention.
FIGS. 6A-6B illustrate various configurations of pistons, motors, and leadscrews according to various embodiments of the present invention.
FIGS. 7A-7H illustrate cross-sectional configurations of pistons and leadscrews according to various embodiments of the present invention.
FIG. 8 illustrates a schematic representation of an implantable pump according to an embodiment of the present invention.
FIG. 9 illustrates a schematic representation of an implantable pump with an external reservoir according to an embodiment of the present invention.
FIG. 10 illustrates a schematic representation of an implantable pump with a fluid port in the piston according to an embodiment of the present invention.
FIG. 11 illustrates a schematic representation of an implantable pump within a casing according to an embodiment of the present invention.
FIG. 12A illustrates a schematic representation of an implantable pump within a casing with a volume transfer bladder according to an embodiment of the present invention.
FIG. 12B illustrates a schematic representation of an implantable pump within a casing with a flexible portion according to an embodiment of the present invention.
FIG. 12C illustrates a schematic representation of an implantable pump casing with a bellows according to an embodiment of the present invention.
FIG. 12D illustrates a schematic representation of an implantable pump casing with two bellows according to an embodiment of the present invention.
FIG. 12E illustrates a schematic representation of an implantable pump casing with a slidable portion according to an embodiment of the present invention.
FIG. 12F illustrates a schematic representation of an implantable pump casing with flexible portions according to an embodiment of the present invention.
FIG. 12G illustrates a schematic representation of an implantable pump casing with a bellows and an elongating balloon according to an embodiment of the present invention.
FIGS. 13A-13B illustrate schematic representations of an implantable pump with an external reservoir according to embodiments of the present invention.
FIG. 14 illustrates a cross-sectional view of a multiple piston implantable pump according to an embodiment of the present invention.
FIG. 15 illustrates a cross-sectional view of a multiple piston implantable pump with a bellows according to an embodiment of the present invention.

### DETAILED DESCRIPTION

The present invention generally provides remotely adjustable gastric banding systems, for example, for treatment of obesity and obesity related conditions, as well as systems for controlling inflation of gastric banding systems.

A remotely adjustable gastric band is a medical device which allows a healthcare worker to adjust a gastric band without utilizing hypodermic needles to connect to an implanted access port. An external, handheld controller can be used to send radio frequency signals for powering and communicating with the implanted device. The implanted device can fill or drain the gastric band as requested by the healthcare worker via the handheld controller. The handheld controller may be a remote device configured to produce a telemetric signal that controls the various components of the gastric banding system.

The filling and draining of the band is accomplished by a set of fluidic elements including pumps, valves, and sensors which monitor and/or move fluid between the gastric band and a reservoir. In accordance with various embodiments, different numbers, types, and orientations of the fluidic elements may be utilized to obtain the desired results. Any and/or all of these various components may be configured to be controlled by a remote transmitter.

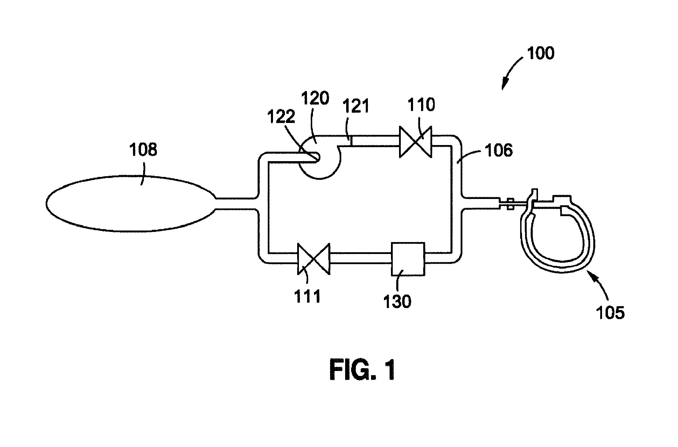

Turning now to FIG. 1, a gastric banding system **100** includes a gastric band **105,** a reservoir **108,** a pump **120,** a sensor module **130** and flow control devices such as valves **110** and **111.** Each of the components of the system **100** is implantable in a patient using conventional surgical techniques. The reservoir **108,** the pump **120,** the valves **110** and **111** and the sensor module **130** may be used to replace or complement a conventional access port for adjusting inflation of the gastric band **105.** In some embodiments, the system **100** includes a conventional access port which can be used, for example, with a hypodermic needle, to fill and drain the gastric band **105.**

The pump **120** and the valves **110** and **111** may move precisely metered volumes of a fluid (e.g., saline, a drug, and/or combinations thereof) from the reservoir **108** through the sensor module **130** into the gastric band **105.** The reservoir **108** may comprise an elastic polymer, a balloon, a rubber container, a silicone container, a collapsible container, a non-elastomeric container, a bellows, and combinations thereof that are configured to contain the fluid.

Moving the fluid into the gastric band **105** causes inflation of at least one bladder, or inflatable member of the gastric band **105** and constricts around the cardia, or upper portion of the stomach, forming a stoma that restricts the passage of food into a lower portion of the stomach. This stoma can provide a patient with a sensation of satiety or fullness that discourages overeating. In contrast, moving the fluid out of at least one inflatable member of the gastric band **105** contracts the pressure around the cardia and allows a stoma to be at least partially released and regains the patient's hunger sensation.

The valves **110** and **111** can be any flow control device to allow precise delivery of fluid and precise flow rates through the valves in response to inductive powering signals received from a remote transmitter. For example, flow control devices as disclosed herein may comprise piezoelectric valves, solenoid valves, membranes, tubes, pressure regulators, ultrasonic flow meters, thermal mechanisms, mass flow sensors, turbines, paddle wheels and combinations thereof.

The pump **120** may comprise any device for moving fluid through a system. For example, the pump **120** may comprise a piezoelectric motor, an electromagnetic motor, an AC motor, a DC motor, a stepper motor and combinations thereof.

Although "transmitter" may be used herein, in should be understood that the remote transmitter may also be a wireless receiver and/or transceiver operable to take readings from the flow sensing module **130** to determine the amount of fluid entering and/or exiting the gastric band **105,** and/or to send or receive other types of information associated with the gastric banding system **100.**

In various embodiments, the remote transmitter provides access to system data and functions and is an external, handheld, reusable battery-powered device. The remote transmitter can be made of any rugged plastic material including, polypropylene, cyclicolephin co-polymer, nylon, and other compatible polymers and the like. Further, the remote transmitter has a user interface including at least one display and at least one user input. The remote transmitter permits a clinician or a patient to navigate through menu driven screens used for data entry, data collection, and control of gastric banding system **100.**

The remote transmitter is capable of communicating with the gastric banding system **100.** "Capable of communicating" as used herein refers to the remote transmitter's ability to establish communications with the gastric banding system **100,** yet still have the ability to break communication and the systems described herein still function. To establish communication, in one example embodiment, once the remote transmitter is initialized, a display shows a searching query for a nearby gastric banding system **100.** As the remote transmitter is brought into range of the gastric banding system **100,** the display shows the strength of the communication link. Once stable communications have been acquired, the display shows the serial number (or other unique patient data) of the system so a clinician can verify they have the appropriate patient records in hand. If the patient requires a tightening of the gastric band **105,** the clinician can enter the amount of the desired volume increase. The remote transmitter can also display the current volume within the gastric band **105** and indicate the new volume as the gastric band **105** fills. The remote transmitter can also indicate desired and actual volumes during the gastric band **105** draining.

In accordance with various embodiments, the gastric banding system **100** allows for a remotely controlled adjustment without needles, non-invasively, by using the remote transmitter. When compared to conventional gastric banding systems having standard access ports which exclusively require syringe access, the presently described systems and apparatus offer several benefits. First, for conventional access ports located under a thick layer of fatty tissue, which is generally the case as the devices are generally used to treat obesity, the access port can be difficult to locate. The present systems reduce or eliminate the need for port location as the use of the remote transmitter removes the necessity of adjustment using a syringe.

Secondly, accessing the access port in conventional systems, when there is ambiguity on its location, can cause damage by accidentally puncturing the tubing which connects the access port to the gastric band. This damage can require another surgery in order to repair the punctured tubing. Further, when a conventional access port cannot be located by palpation, x-ray imaging may be required to guide a needle into the access port. Such imaging practices put a patient at risk for x-ray radiation exposure. The present systems and apparatus remove the need for these unnecessary procedures and save the patient from x-ray radiation exposure. As described herein, the present systems and apparatus are compatible with magnetic resonance imaging (MRI), which is much safer for a patient.

The fluids used within the systems include any fluid that is biocompatible and incompressible. The fluid has no adverse effect on the patient in the unlikely event that a leak emanates from the system. The fluid can simply be water or any biocompatible polymer oil such as caster oil. In an example embodiment, the fluid is saline, a drug, and/or combinations thereof.

The tubing **106** connects the various components of the system **100** and comprises any biocompatible flexible tubing that does not degrade *in vivo.* The tubing **106** is configured to withstand hydraulic forces up to hundreds of psi without leakage. This hydraulic pressure tolerance is true of the entire fluid path of the systems described herein. Although the systems described herein do not generally leak, if they do, fluid is generally lost at a rate less than about 0.2cc/yr, for example, between about 0.01 to 0.07 cc/yr.

According to various embodiments, components of the gastric banding system **100** may be placed in their respective positions within a patient using common surgical techniques. The surgical techniques may be similar to those used in the placement of conventional gastric banding systems. For example, the gastric band **105** may be placed around the stomach using laparoscopic techniques, as known to those of skill in the art. Like a conventional access port, various components of the gastric banding system **100** may be sutured onto the rectus muscle sheath or any other conveniently accessible muscle. The tubing **106** to the gastric band **105** passes through the rectus muscle into the peritoneal cavity in the same manner as the tubing of a conventional access port.

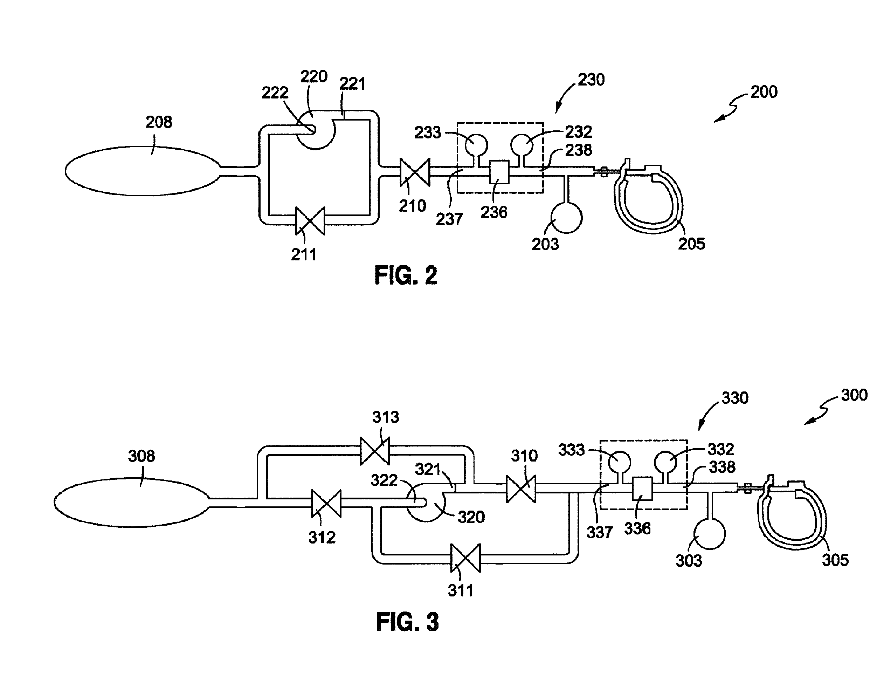

FIG. 2 illustrates an embodiment of the gastric banding system **200** which comprises a valve **211** and a pump **220** connected in parallel to each other. The parallel combination of the valve **211** and the pump **220** is connected in serial with a reservoir **208** on one end and with a valve **210** on the other end. The sensor module **230** is connected in serial with the valve **210** and is disposed between the valve **210** and the gastric band **205.**

The sensor module **230** comprises two pressure sensors, flow meters **232** and **233,** and/or other devices for measuring a desired property or parameter of the fluid in the gastric banding system **200.** For example, the sensor module **230** may be configured to measure flow speed/rate, pressure, fill volume, stress, strain, linear position and combinations thereof.

A calibrated orifice **236** is disposed between the flow meters **232** and **233.** The sensor module **230** comprises a first inlet/outlet **237** and a second inlet/outlet **238.** Because fluid may flow in both directions within the gastric banding system **200,** the inlets/outlets **237** and **238** may alternately function as inlets and outlets. The valve **210** is coupled to the inlet/outlet **237** for controlling the fluid flow into or out of the gastric band **205.**

Although a remote transmitter may be utilized to control various components of the gastric banding system **200,** should the remote transmitter be unavailable, damaged, out of power, or in the event of an emergency, an adjustment of the gastric band **205** may be performed invasively using a needle. For example, an access port **203** (commonly used in other gastric banding systems) may be included in the gastric banding system **205,** in addition to the other components illustrated in FIG. 2. The access port may be located between the valve **210** and the gastric band **205.**

Thus, a clinician may choose to use a standard needle for adjustments, for example, if any of the electronics associated with the gastric banding system **200** become inoperable. Even if the electronics are unavailable, the access port would be available to adjust the gastric band **205.** In the unlikely event that the access port is used, it may be located away from the tubing connection to the gastric band **205** to reduce the potential for tubing needle sticks. Information regarding hydraulically adjustable gastric banding systems including subcutaneous fluid access ports/injection ports may be found in Vincent, U.S. Patent No. 5,601,604; Kusmack, U.S. Patent No. 5,226,429; Birk, U.S. Patent Application Publication No. 2005/0192531, the disclosure of each of these patents and publications is being incorporated herein in its entirety by this specific reference.

In embodiments as illustrated in FIGS. 2 and 3, it is possible to measure the fluid flow rate during the process of pumping. The pulsatile flow of pumps may produce a quickly varying pressure wave which can affect the accuracy of pressure and flow measurements. This high pressure gradient can be reduced and the measurement accuracy can be increased by rearranging existing elements and placing additional elements within the flow path such as a valve membrane, soft tubing, or a specially designed fluidic pressure regulator.

Various embodiments of the present invention are configured to speed up the medical procedure of increasing the volume or pressure within a gastric band. The various configurations also facilitate decreasing the time required to perform a partial or complete draining of the gastric band. Thus, embodiments of the present invention provide an implantable device that has an increased efficiency of filling and draining the inflatable portion of the gastric band, with respect to existing implantable pumps.

With continued reference to FIG. 2, in an embodiment, the flow sensing module **230** functions both in pumping and draining the gastric band **205** to facilitate measuring the fluid flow rate in both directions. For example, the flow sensing module **230** may comprise the pressure sensors **232** and **233** positioned on opposite sides of the calibrated orifice **236.** When the calibrated orifice **236** is intended to operate during both the filling and draining process, the calibrated orifice **236** selected may be designed to accurately control bidirectional fluid flow. In various embodiments, the flow sensing module **230** and/or the gastric banding system **200** may include ultrasonic flow meters, thermal mechanisms such as mass flow sensors, or mechanical systems such as turbines and paddle wheels.

An embodiment as illustrated in FIG. 2 allows the pump **220** to "warm up" or stabilize before proceeding with the transfer of fluid between the reservoir **208** and the gastric band **205.** For example, the valve **211** connects a pump outlet **221** to a pump inlet **222,** and the valve **211** may be opened while the valve **210,** to the flow sensing module **230** and the gastric band **205,** remains closed. Opening the valve **211** in this manner allows the pump **220** to stabilize. After stabilization, the valve **210** may be opened. Allowing the pump **220** to stabilize before transferring fluid through the flow sensing module **230** facilitates more accurate measurements of the flow conditions of the fluid. Similarly, in an embodiment as illustrated in FIG. 3, the valves **312** and **313** may be opened before the valves **310** and **311** are opened so that the pump **320** may stabilize before the fluid is directed through the flow sensing module **330** and into the gastric band **305.** The valves **312** and **313** may be configured to withstand approximately 30 psi.

With reference to FIG. 3, an embodiment of the gastric banding system **300** may be configured to modify the drain rate of the gastric band **305** using the passive and active properties of the pump **320.** For example, opening valves **310** and **312** while keeping valves **311** and **313** closed, causes fluid to flow from the gastric band **305** through the deenergized pump **320** to the reservoir **308.** This fluid path through the deenergized pump **320** significantly reduces the drain rate from the gastric band **305.** Additionally, by opening valves **311** and **313** while keeping valves **310** and **312** closed, and directing the drainage through the deenergized pump **320** in a different direction, the drain rate can be slightly reduced since the one-way valves favor this direction of flow.

Further, opening valves **311** and **313** while keeping valves **310** and **312** closed, and supplying voltage to the pump **320,** the drain rate can be slightly increased. As the voltage applied to the pump **320** is increased, the drain flow rate from the gastric band **305** to the reservoir **308** is also increased. This property may be advantageous, for example, when the gastric band **305** contains too little pressure to drain at a desired rate.

In accordance with various embodiments, for example, as illustrated in FIGS. 2 and 3, the gastric banding systems **200** and **300** are configured to obtain an overall lower leak rate because of their arrangement. For designs where the flow sensing module **230** and **330** is determined to contribute to the system leak rate, the arrangement may be adjusted by placing a valve between the flow sensing modules **230** and **330** and the gastric bands **205** and **305.** Such embodiments facilitate reducing the flow rate from the various pumps because the fluid is pumped through the flow resistance caused by the flow sensing modules **230** and **330.**

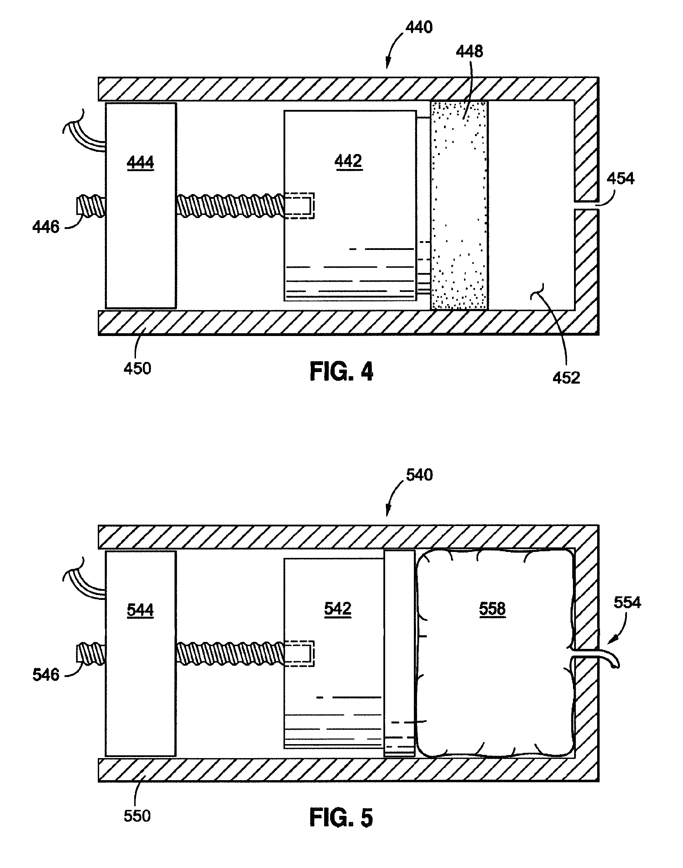

In various embodiments, certain components of the gastric banding system may be combined into a single device. With reference to FIG. 4, one embodiment comprises an implantable pump **440** that includes an integrated chamber or reservoir **452** configured to contain a fluid. The reservoir **452** may comprise an elastic polymer, a balloon, a rubber container, a silicone container, a collapsible container, a non-elastomeric container, a bellows, and combinations thereof that are configured to contain the fluid. The pump **440** further comprises a motor **444** that drives a piston **442** via a leadscrew **446** to cause fluid to exit and/or enter a chamber **452** via a fluid port **454.** Thus, the pump **440** may be referred to herein as an implantable, motorized piston pump. It should be understood that the leadscrew **446** may comprise a screw, a rod, combinations thereof and/or other structure capable of driving the piston **442** using the motor **444.** For example, the leadscrew **446** may comprise a smooth portion and a textured portion to facilitate moving the piston **442** by translational movement.

Although the pump **440** may be utilized in connection with filling and draining a gastric band, it should be understood that the pump **440** has other uses contemplated within the scope of this disclosure. For example, there are many medical situations where the pump **440** may be employed, where it is useful to move the fluid from one place within the body to another place within the body, such as in drug delivery and the modulation of artificial sphincters.

The motor **444** uses electrical energy to turn a gear which moves the leadscrew **446** back and forth to move the piston **442.** The piston **442** and a seal **448** are tightly coupled to and housed within a block **450.** The space bounded by the piston **442** and the interior of the block **450** forms a chamber **452,** the volume of which contracts and expands as the piston **442** moves within the block **450.**

Various types of motors may be used to drive the piston **442,** each having various electrical requirements, driving forces, driving speeds and compatibility with magnetic resonance imaging. The motor **444** may comprises any system or device capable of developing linear and/or rotary motion. Linear motors such as piezoelectric linear motors or electromagnetic linear motors may be used to directly drive the piston. For example, an armature may be used to move the piston **442,** which does not have the helical shape of the leadscrew **446.** In an embodiment, a non-electromagnetic and/or non-magnetic linear drive motor may be used.

Further, in various embodiments, a rotary drive motor may be geared to provide the desired linear motion. Rotary motors such as AC, DC, stepper, or piezoelectric motors can be used to drive a gear system which, in turn, moves the piston **442.** The gear system may be any style which is capable of converting rotational motion into linear motion, and examples include a leadscrew, a ball screw, a jack screw, a rod, and combinations thereof. In various embodiments, the leadscrew **446** may rotate and/or translate with respect to the outer casing of the motor **444** since the gear system may be located between the motor **444** and the leadscrew **446** or between the leadscrew **446** and the piston **442.**

The piston **442** is configured to use the motion of the leadscrew **446** to apply force on the fluid in the reservoir **452.** The piston **442** may be of any cross-sectional shape, including circular, annular, triangular and/or other shapes. The piston **442** may act directly on the fluid by utilizing a seal **448** and/or a wiper.

With reference to FIG. 5, in one embodiment, a piston **542** may act indirectly on the fluid if it pushes on a container **558** such as an elastic balloon, an inelastic bladder, a bellows, and combinations thereof. If the piston acts on the container **558,** a seal may not be required and/or the piston may act on the container to move the fluid without a seal. However, a seal may be utilized in connection with the container **558.**

With reference to FIGS. 4 and 5, the piston **442 (542)** and/or the seal **448** may have a flat face or a tapered face depending on the desired flow properties and the available space for pump **440 (540).** Flow properties and/or device size may also determine if the fluid port **454 (554)** is placed within the block **450 (550)** in-line with the center of piston **442 (542)** or off-axis from the center of piston **442 (542).** Additionally, as will be discussed further below, fluid port **454 (554)** may be placed within piston **442 (542)** to obtain a different set of operating parameters and/or properties.

It should be understood that although the various figures may illustrate a gap and/or space between the piston and the block and/or between the seal and the block, the various components of the pumps disclosed herein are configured to pump a fluid into a gastric band. Thus, the pistons and/or seals are configured to move within the pump in a manner that causes the fluid to be expelled from the reservoir. Tolerances may be tighter or looser than those illustrated in the figures without departing from the scope of the present invention. Further, other tolerances, materials, seals, lubricants, components and/or combinations thereof may be utilized to obtain the desired pumping characteristics. Additionally, in some embodiments, the pump may not include a seal between the piston and the filling reservoir.

With reference again to FIG. 4, in an embodiment, the orientation or configuration of the motor **444** and the piston **442** may affect function and/or size of the pump **440.** For example, when the motor **444** is placed inline with the piston **442,** the load of the piston **442,** which is inline with the motor **444,** produces a symmetrical distribution of forces. An alternate embodiment includes a configuration where the motor **444** is placed off axis to the piston **442.** This configuration results in an asymmetric loading of the piston **442.**

The motor **444,** the gearing, and the leadscrew **446** can be connected to the piston **442** in a way to match certain characteristics to the mechanical system by pulling and/or pushing the piston **442** or to obtain substantially equal pushing and pulling forces. In some motor/piston combinations, a return spring may be utilized to assist the movement of the piston **442** into or out of the block **450.**

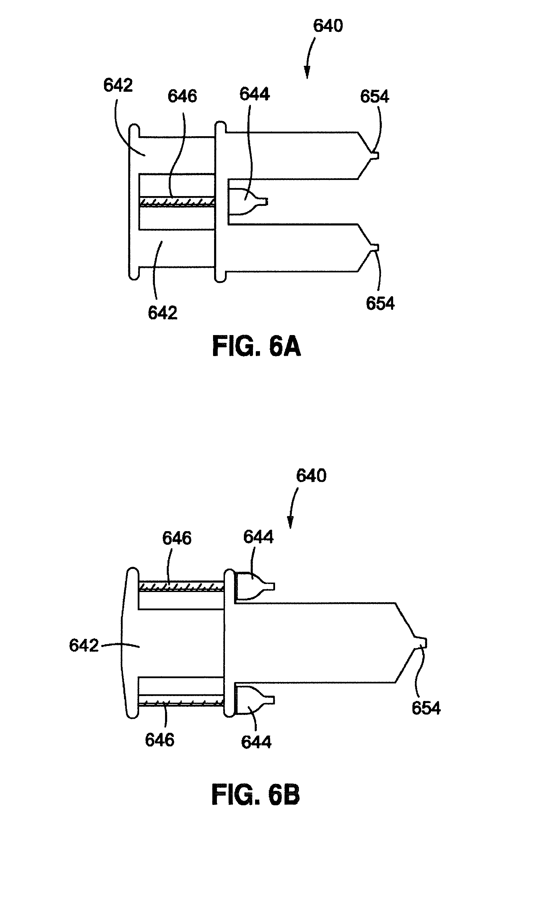

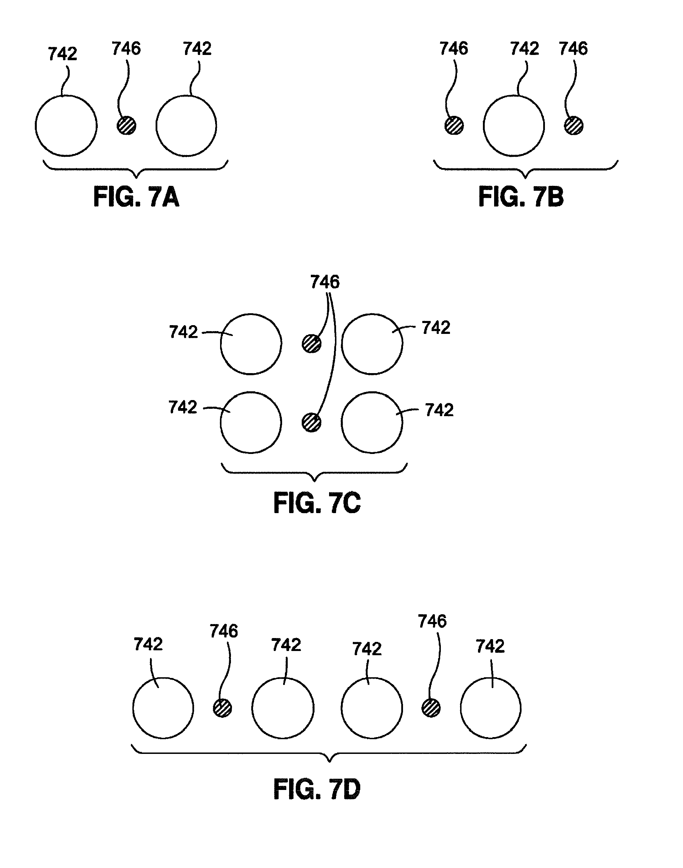

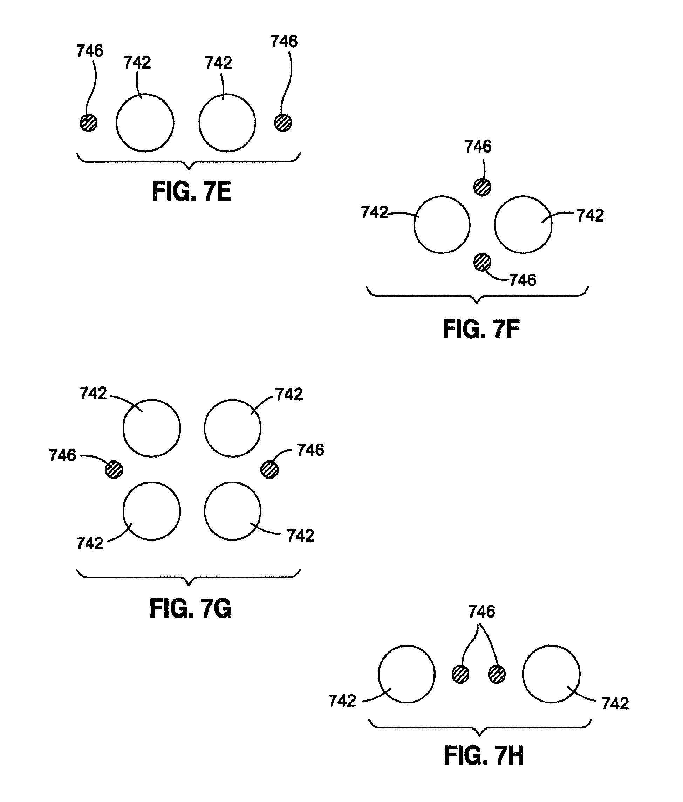

Although FIG. 4 illustrates the pump **440** having one motor and one piston, it should be understood that any number and/or combination of motors and pistons may be utilized in accordance with the present invention. For example, FIG. 6A illustrates an embodiment with two pistons **642** and one motor **644** and one leadscrew **646.** FIG. 6B illustrates an embodiment with one piston **642** and two motors **644** and two leadscrews **646.** FIGS. 7A-7H illustrate various other combinations of the pistons **742** and the leadscrews **746** according to embodiments of the present invention.

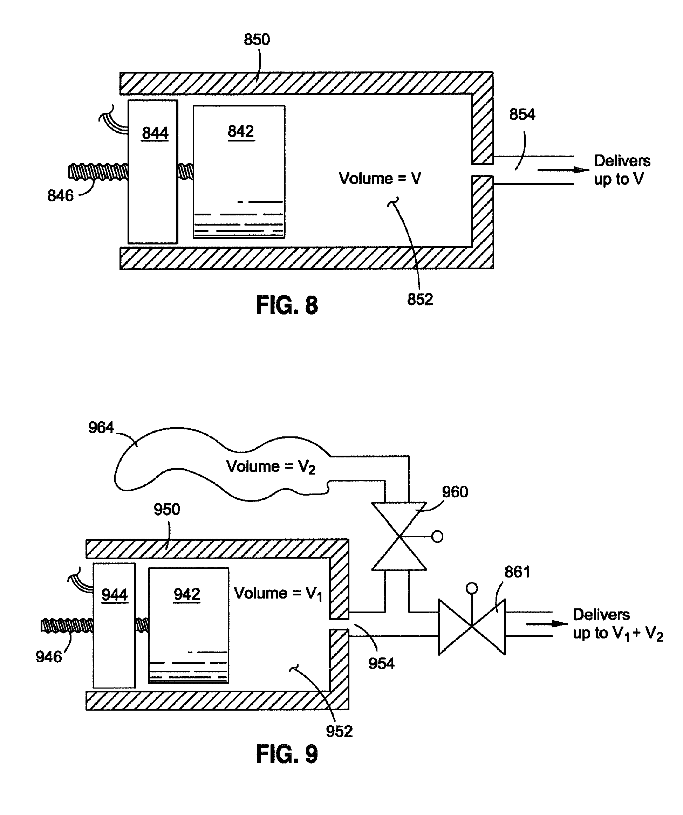

With reference to FIG. 8, in one embodiment, the total volume (V) which is moved by the piston **842** may reside within the reservoir **852.** In this case, the volume pumped by the system may be any part of the total volume V, but not more than V.

However, with reference to FIG. 9, one embodiment facilitates delivering more than the volume (V₁) of fluid contained in the reservoir **952.** For example, flow control devices such as valves **960** and **961** and the external reservoir **964** having a volume (V₂) may be added to the system, and the total amount which can be delivered (e.g., V₁ + V₂) can be greater than the volume of the reservoir **952.** The reservoir **952** and/or the external reservoir **964** may comprise an elastic polymer, a balloon, a rubber container, a silicone container, a collapsible container, a non-elastomeric container, a bellows and combinations thereof that are configured to contain the fluid. In one embodiment, the reservoir **952** has a volume of between approximately 0.1-5 cc (preferably approximately 1 cc), and the external reservoir **964** has a volume of between approximately 1-10 cc (preferably approximately 5 cc).

With the valve **960** open and the valve **961** closed, the piston **942** can reciprocate within the block **950** in order to drain or fill the external reservoir **964.** With the valve **960** closed and the valve **961** open, the piston **942** can reciprocate within the block **950** in order to drain or fill the gastric band. By placing the valves **960** and **961** proximate to the inlet/outlet port **954** and reducing the amount of fluid between the valves and the fully compressed piston **942,** the dead space of the system can be reduced and the efficiency of the system can be improved. By a similar method, or by increasing the stroke volume, the compression ratio (volume pumped in one stroke divided by the dead space volume) can be increased.

In accordance with various embodiments, the motorized piston pump system is reversible. For example, with reference to FIG. 8, when the total volume is contained within the reservoir **852,** fluid may be driven out of the reservoir **852** by driving the piston **842** through the block **850** toward the inlet/outlet **854,** and the fluid can be returned into the reservoir **852** by pulling the piston **842** out of the block **850** away from the inlet/outlet **854.**

With reference to FIG. 9, reversing the flow of fluid may also be accomplished. The valves **960** and **961** and the piston **942** may be sequenced, as discussed above, so that the fluid flows into or out of the reservoir **952.**

Although various embodiments disclosed herein comprise a motor that is located outside of the fluid path which it is used to pump (see, e.g., FIG. 4 where piston **442** pumps the fluid in the reservoir **452).** In an embodiment, the motor may be configured to function within a fluid environment, and may be positioned in contact with or surrounded by the fluid.

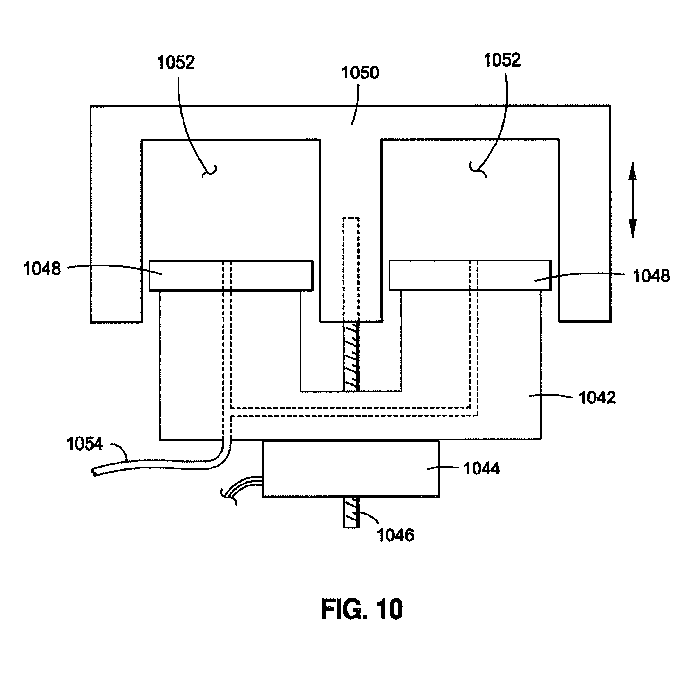

With reference to FIG. 10, in one embodiment, the inlet/outlet port **1054** may be placed within the piston **1042,** as compared to within the block **950** as illustrated in FIG. 9. The location of the port within the piston or the block may be determined by certain performance requirements. For example, if the fluid connection(s) is/are routed out of the same assembly that holds the motor, both the fluid connection(s) and the electrical connection(s) to the motor can remain stationary and be less susceptible to bending fatigue, as illustrated in FIG. 10.

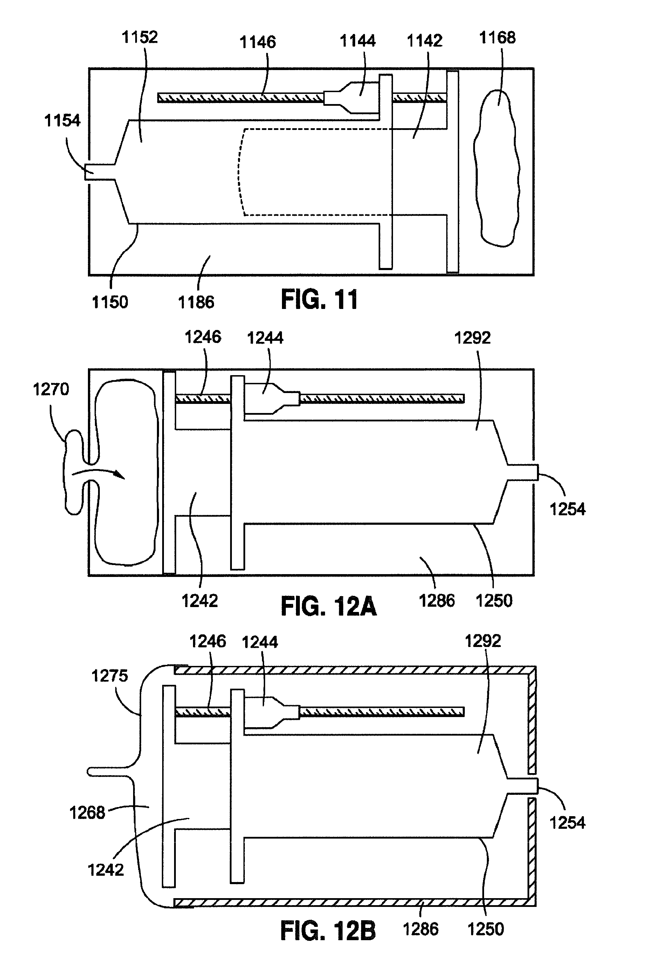

Turning now to FIG. 11, when an internal element (such as a piston **1142)** moves within a casing **1186,** the friction or resistive forces may be reduced by interposing low friction or lubricious materials between the moving surfaces. Additionally, the friction may be reduced by including fluid- or air-filled spaces between the moving surfaces of the pump and casing components.

As the piston **1142** moves within the enclosed, sealed casing **1186** and the block **1150** and ejects fluid from the reservoir **1152,** the overall volume of the system decreases. However, because the casing **1186** is a rigid housing with a fixed volume, the discrepancy results in a mismatched vacuum in vacuum area **1168** of the casing **1186.** In order to compensate for this volume/vacuum mismatch condition, various embodiments comprise one or more of a flexible housing, an internal equalizer, or a reduced volume change in the piston block **1150** and/or in casing **1186.** For example, a rigid housing may be converted to a flexible housing and then any changes in the internal volume will cause corresponding volume changes within the housing.

FIGS. 12A-12G illustrate various embodiments for adapting an external housing **1250** to include flexible structures. The flexible structures can be constructed either from metal or polymeric material. For example, FIG. 12A illustrates a volume transfer bladder **1270** that is partially located within and without the casing **1286.** As the piston **1242** moves in the block **1250,** the volume transfer bladder **1270** enters or exits the casing **1286** to compensate for the change in volume. Similarly, as illustrated in FIG. 12B, part of the casing **1286** may comprise a flexible portion **1275** that expands and contracts as the piston **1242** moves within the block **1250.**

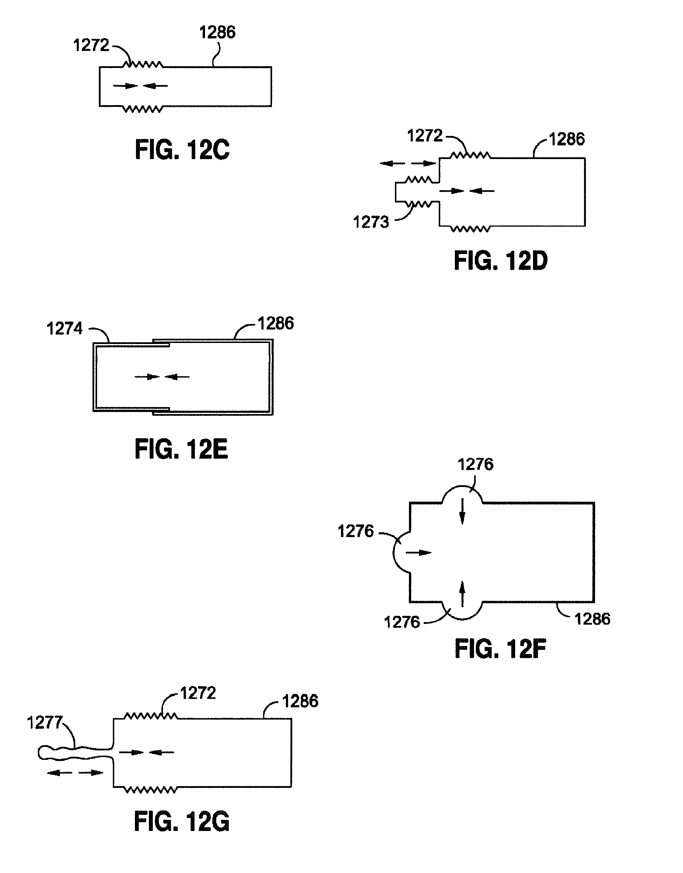

With reference to FIG. 12C, in one embodiment, the overall length of the casing **1286** may be configured to change to compensate for the change in volume. For example, a first set of bellows **1272** may be utilized to facilitate the change in length of the casing **1286.** With reference to FIG. 12D, the first set of bellows **1272** may be used in conjunction with a second set of bellows **1273** that moves in opposition to the first set of bellows **1272.** Such a configuration allows for compensation of a volume change in the casing **1286** while maintaining a fixed length for the casing **1286.** Furthermore, with reference to FIG. 12G, the first set of bellows **1272** may be utilized in connection with an elongating balloon **1277** that extends from or retracts into the casing **1286** depending on the motion of the piston.

With reference to FIG. 12E, one embodiment includes a sliding segment **1274** that slides with respect to the casing **1286** in response to motion of the piston. Such motion allows the volume of the casing **1286** to change as fluid exits or enters the reservoir of the pump. Utilizing sliding components may improve the system's resistance to fatigue and failure. However, in some embodiments, for example, with reference to FIG. 12F, the casing **1286** may comprise at least one flexible segment **1276** that expands or contracts in response to motion of the piston.

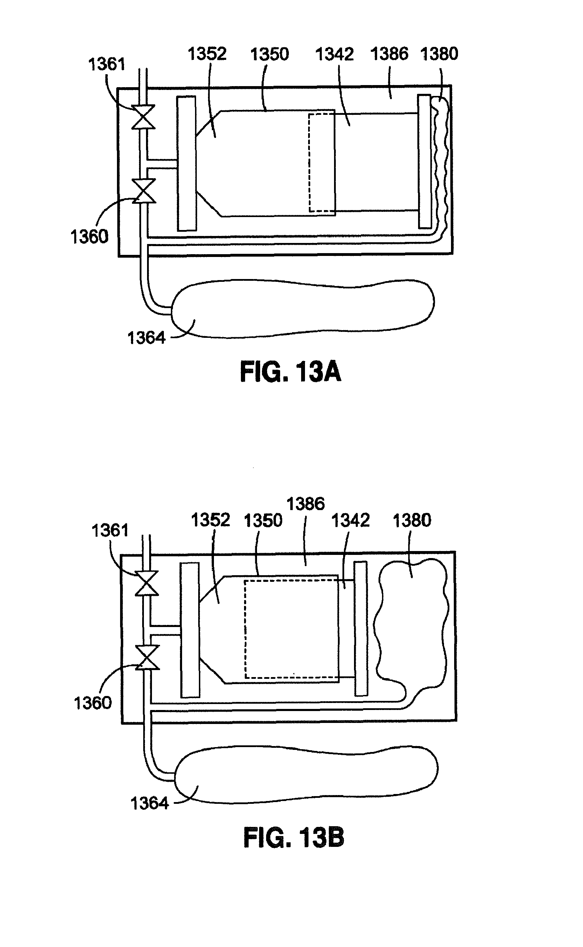

In accordance with another embodiment, and with reference to FIGS. 13A-13B, an internal equalizer mechanism may be used to compensate for a change in volume. For example, the casing **1386** may comprise a bladder **1380** with a volume that increases or decreases as the piston **1342** moves within the block **1350.** The bladder **1380** may be utilized with or without an external reservoir **1364.** The valves **1360** and **1361** may be alternately opened or closed to control the volume of fluid within the reservoir **1352** and/or the external reservoir **1364,** as discussed above with respect to FIG. 9, to compensate for volume changes due to movement of the piston **1342.** When the valve **1361** is opened, fluid may be expelled from the reservoir **1352,** and the volume change caused by movement of the piston **1342** causes fluid from the external reservoir **1364** to be drawn into the bladder **1380** (see FIG. 13B). Then, if more fluid is desired within the reservoir **1352,** the valve **1361** may be closed and the valve **1360** may be opened to allow fluid to be drawn from the bladder **1380** and/or the external reservoir **1364.**

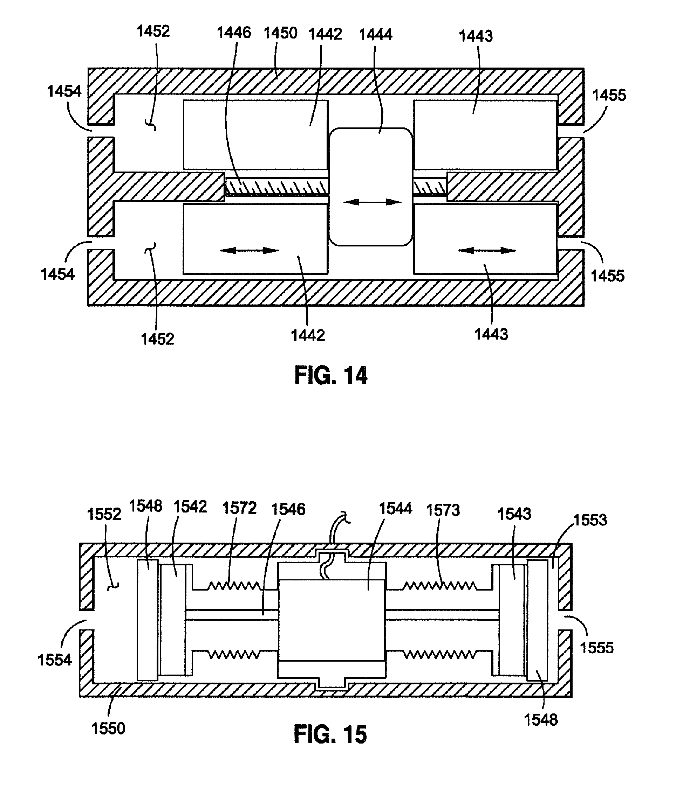

Another embodiment configured to compensate for the vacuum/pressure mismatch is illustrated in FIG. 14, for example, to minimize the parasitic effects of a vacuum. Such an embodiment comprises multiple, opposing pistons **1442** and **1443** that are configured to reduce the volume/pressure mismatch by minimizing and/or eliminating the amount of volume change in the motor piston pump. For example, as the piston **1442** moves toward the inlet/outlet **1454,** the piston **1443** moves away from the inlet/outlet **1455** to compensates for the volume change induced by the movement of the piston **1442.** By arranging two or more pistons **1442** and **1443** to act oppositely for a given motor **144** stroke (e.g., one pumping and one filling), a nearly continuous pumping action may be achieved. The inlet/outlet **1455** may be connected to an external reservoir as illustrated in FIGS. 13A-13B, and may operate in conjunction with various valves (also as illustrated in FIGS. 13A-13B) to facilitate movement of the fluid within the system. The opposing pistons **1442** and **1443** facilitate reducing the overall size of the implantable device.

With reference to FIG. 15, one embodiment comprises sealed bellows **1572** and **1573** that expand and contract in response to movement of the pistons **1542** and **1543.** The bellows **1572** and **1573** may be constructed of metal or any other material that facilitates operation of the pump as disclosed herein. By enclosing the motor **1544** and the leadscrew **1546** within the bellows **1572** and **1573,** the rotating parts and electronics may be hermetically sealed. When two opposing bellows **1572** and **1573** are moved opposite of one another (i.e., one compresses as the other expands), the pressure of the gas or fluid within the sealed system can be kept substantially constant. The seals **1548** (and/or bladders as discussed with respect to FIG. 5-flexible bladder **558)** may be used to further seal the motor **1544** and/or other electronics or components of the piston pump.

Unless otherwise indicated, all numbers expressing quantities of ingredients, volumes of fluids, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, certain references have been made to patents and printed publications throughout this specification. Each of the above-cited references and printed publications are individually incorporated herein by reference in their entirety.

Specific embodiments disclosed herein may be further limited in the claims using consisting of or and consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

### FURTHER EMBODIMENTS

1. An implantable device for filling and draining an inflatable portion of a gastric band, the implantable device comprising:
   a reservoir for holding a fluid;
   a sensor positioned between the gastric band and the reservoir, the sensor having a first inlet and a second inlet, the fluid flowing from the first inlet to the second inlet when filling the inflatable portion of the gastric band, the fluid flowing from the second inlet to the first inlet when draining the inflatable portion of the gastric band, the sensor monitoring a parameter of the fluid when filling and draining the inflatable portion of the gastric band;
   a first flow control device, coupled to the first inlet of the sensor, for controlling a flow of the fluid when filling and draining the inflatable portion of the gastric band; and
   a pumping device, coupled between the reservoir and the first flow control device, for moving the fluid into and out of the inflatable portion of the gastric band, the pumping device capable of being activated and deactivated using a telemetric signal received from a remote device.
2. The implantable device of 1 wherein the sensor includes two pressure sensors and a calibrated orifice positioned fluidly between the two pressure sensors.
3. The implantable device of 1 wherein the first flow control device is selected from a group consisting of a valve, a membrane, a tube, a pressure regulator, an ultrasonic flow meter, a thermal mechanism, a mass flow sensor, a turbine, a paddle wheel, and combinations thereof.
4. The implantable device of 1 further comprising a second flow control device coupled to the second inlet of the sensor for controlling movement of the fluid when draining the gastric band.
5. The implantable device of 4 wherein the second flow control device is selected from a group consisting of a valve, a membrane, a tube, a pressure regulator, an ultrasonic flow meter, a thermal mechanism, a mass flow sensor, a turbine, a paddle wheel, and combinations thereof.
6. The implantable device of 1 wherein the pumping device is selected from a group consisting of a pump, a piezoelectric motor, an electromagnetic motor, an AC motor, a DC motor, a stepper motor, and combinations thereof.
7. The implantable device of 1 wherein the parameter is selected from a group consisting of a flow speed, a pressure, a fill volume, a stress, a strain, a linear measurement, and combinations thereof.
8. The implantable device of 1 wherein the reservoir is selected from a group consisting of an elastic polymer, a balloon, a rubber container, a silicone container, and combinations thereof.
9. The implantable device of 4 further comprising an access port fluidly coupled between the second flow control device and the inflatable portion of the gastric band.
10. The implantable device of 1 wherein the fluid is selected from a group consisting of a drug, a saline solution, and combinations thereof.
11. The implantable device of 1 wherein the pumping device comprises a non-magnetic electrical drive.
12. An remotely adjustable gastric banding system comprising:
   a gastric band having an inflatable portion;
   a reservoir for holding a fluid for filling and draining the inflatable portion of the gastric band;
   flexible tubing connecting the reservoir to the inflatable portion of the gastric band;
   a sensor module positioned between the gastric band and the reservoir, the sensor module comprising:
      a first inlet;
      a second inlet;
      a first flow meter coupled to the first inlet;
      a second flow meter coupled to the second inlet; and
      a calibrated orifice disposed between the first flow meter and the second flow meter, the fluid flowing from the first inlet to the second inlet when filling the inflatable portion of the gastric band, the fluid flowing from the second inlet to the first inlet when draining the inflatable portion of the gastric band, the sensor module monitoring a parameter of the fluid when filling and draining the inflatable portion of the gastric band;
   a first valve, coupled to the first inlet of the sensor module, for controlling a flow of the fluid when filling and draining the inflatable portion of the gastric band;
   a piezoelectric pump coupled between the reservoir and the first valve, for moving the fluid into and out of the inflatable portion of the gastric band, the piezoelectric pump capable of being activated and deactivated using a telemetric signal received from a remote device, the pumping device comprising a non-magnetic electrical drive;
   a second valve coupled in parallel with the piezoelectric pump, wherein the second valve and the piezoelectric pump are coupled to the first valve and the reservoir, wherein the first valve remains closed while the second valve is opened to allow the piezoelectric pump to stabilize before opening the first valve to facilitate filling and draining the gastric band; and
   an access port fluidly coupled between the second flow control device and the inflatable portion of the gastric band.
13. The remotely adjustable gastric banding system of 12, further comprising a third valve disposed between the second inlet of the sensor module and the gastric band to facilitate reducing a leak rate of the remotely adjustable gastric banding system.
14. An implantable, motorized device that facilitates movement of fluid to an inflatable portion of a gastric band, the implantable, motorized device comprising:
   a housing having a fluid port;
   an integrated reservoir positioned within the housing and adjacent to the fluid port and capable of holding the fluid;
   a first piston positioned within the housing and adjacent to the integrated reservoir, the first piston capable of moving a portion of the fluid from the integrated reservoir to the inflatable portion of the gastric band;
   a first leadscrew coupled to the first piston and causing the first piston to move between a first position and a second position; and
   a motor attached to the housing and coupled to the first leadscrew, the motor moving the first piston between the first position and the second position, and when the first piston is in the first position, the integrated reservoir having a larger volume than when the first piston is in the second position, the motor being controlled by a telemetric signal received from a remote device.
15. The implantable, motorized device of 14 wherein the integrated reservoir is selected from a group consisting of a balloon, an elastic polymer, a flexible bladder, a rubber container, a silicone container, and combinations thereof.
16. The implantable, motorized device of 14 further comprising an external reservoir, coupled to the fluid port, for filling the integrated reservoir with additional fluid.
17. The implantable, motorized device of 14 wherein the external reservoir is selected from a group consisting of a balloon, an elastic polymer, a flexible bladder, a rubber container, a silicone container, a non-elastomeric container, a bellows, and combinations thereof.
18. The implantable, motorized device of 14 wherein the motor comprises a non-magnetic electrical drive or a magnetic motor.
19. The implantable, motorized device of 14 further comprising a first flow control device coupled to the fluid port for controlling movement of the fluid when filling the gastric band.
20. The implantable, motorized device of 19 wherein the first flow control device is selected from a group consisting of a valve, a tube, and a regulator, and combinations thereof.
21. The implantable, motorized device of 19 further comprising a second flow control device coupled to the external reservoir for controlling movement of the fluid when filling the gastric band.
22. The implantable, motorized device of 14 wherein the fluid is selected from a group consisting of a drug, a saline solution, and combinations thereof.
23. The implantable, motorized device of 14 wherein the motor moves the first leadscrew by rotational movement.
24. The implantable, motorized device of 14 wherein the first leadscrew comprises a rod and the motor moves the rod by translational movement.
25. The implantable, motorized device of 14 further comprising a seal connected to the first piston and positioned between the first piston and the integrated reservoir.
26. The implantable, motorized device of 14 further comprising a slidable seal connected to the first piston, wherein the slidable seal maintains a seal with the housing as the first piston moves from the first position to the second position.
27. The implantable, motorized device of 14 wherein the first piston is positioned between the first leadscrew and the integrated reservoir.
28. The implantable, motorized device of 14 further comprising a second leadscrew coupled to the motor.
29. The implantable, motorized device of 14 further comprising a second piston coupled to the first leadscrew.
30. The implantable, motorized device of 14 wherein the housing is made of a material selected from a group consisting of a rigid material and a flexible material.
31. The implantable, motorized device of 14 wherein the housing comprises a plurality of housing walls defining boundaries of the integrated reservoir.
32. The implantable, motorized device of 14 wherein the housing defines a chamber for housing the integrated reservoir, the first piston, the first leadscrew, and the motor.
33. The implantable, motorized device of 14 wherein the piston does not include a seal.
34. An implantable, motorized piston pump that facilitates movement of fluid to an inflatable portion of a gastric band, the implantable, motorized piston pump comprising:
   a housing having a fluid port;
   an integrated reservoir positioned within the housing and adjacent to the fluid port and capable of holding the fluid, wherein the integrated reservoir comprises a container having a volume of between approximately 0.1-5 cc, and wherein the container is attached to the fluid port;
   a piston positioned within the housing and adjacent to the integrated reservoir, the piston capable of moving a portion of the fluid from the integrated reservoir to the inflatable portion of the gastric band, wherein the piston is capable of moving the portion of the fluid without a seal;
   a leadscrew coupled to the piston and causing the piston to move between a first position and a second position;
   a motor attached to the housing and coupled to the leadscrew, the motor moving the piston between the first position and the second position, and when the piston is in the first position, the integrated reservoir having a larger volume than when the piston is in the second position, the motor being controlled by a telemetric signal received from a remote device;
   an external reservoir, coupled to the fluid port, for filling the integrated reservoir with additional fluid, wherein the external reservoir has a volume of between approximately 1-10 cc;
   a first valve coupled to the fluid port for controlling movement of the fluid when filling or draining the gastric band;
   a second valve coupled to the external reservoir for controlling movement of the fluid when filling or draining the integrated reservoir, wherein the fluid flows between the external reservoir and the integrated reservoir as the piston reciprocates in the housing when the first valve is closed and the second valve is opened; and
   a bladder disposed in the housing and coupled to the external reservoir, wherein the bladder increases in volume as the piston moves from the first position to the second position to compensate for the integrated reservoir having a smaller volume than when the piston is in the first position.
35. The implantable, motorized piston pump of 34 wherein the fluid flows between the integrated reservoir and the gastric band as the piston reciprocates within the housing when the second valve is closed and the first valve is opened.
36. The implantable, motorized piston pump of 34 wherein the housing comprises a flexible segment to compensate for a vacuum/pressure mismatch.
37. An implantable, motorized device that facilitates movement of fluid to an inflatable portion of a gastric band, the implantable, motorized device comprising:
   a housing having a first fluid port and a second fluid port;
   a first reservoir positioned within the housing and adjacent to the first fluid port and capable of holding a first portion of the fluid;
   a second reservoir positioned within the housing and adjacent to the second fluid port and capable of holding a second portion of the fluid;
   a first piston positioned within the housing and adjacent to the first reservoir, the first piston capable of moving the first portion of the fluid out of the first reservoir into the inflatable portion of the gastric band;
   a second piston positioned within the housing and adjacent to the second reservoir, the second piston capable of moving the second portion of the fluid into the second reservoir;
   a bellows disposed between the first piston and the second piston; and
   a motor located within the bellows and coupled to the first piston and the second piston to facilitate moving the first piston and the second piston within the housing, wherein the bellows is hermetically sealed and expands and contracts as the first piston and the second piston move within the housing.
38. The implantable, motorized device of 37 wherein the first piston and the second piston act oppositely during a stroke of the motor to facilitate a nearly continuous pumping action.
39. The implantable, motorized device of 37 wherein the first piston moves the first portion of the fluid out of the first reservoir and the second piston moves the second portion of the fluid into the second reservoir during a stroke of the motor.
40. The implantable, motorized device of 37 wherein the first piston and the second piston are configured to reduce the overall size of the implantable, motorized device.
41. The implantable, motorized device of 37 wherein the first piston and the second piston are configured to minimize the parasitic effects of a vacuum.

## Claims

1. An implantable, motorized device that facilitates movement of fluid to an inflatable portion of a gastric band, the implantable, motorized device comprising:
a housing having a fluid port;
an integrated reservoir positioned within the housing and adjacent to the fluid port and capable of holding the fluid;
a first piston positioned within the housing and adjacent to the integrated reservoir, the first piston capable of moving a portion of the fluid from the integrated reservoir to the inflatable portion of the gastric band;
a first leadscrew coupled to the first piston and causing the first piston to move between a first position and a second position; and
a motor attached to the housing and coupled to the first leadscrew, the motor moving the first piston between the first position and the second position, and when the first piston is in the first position, the integrated reservoir having a larger volume than when the first piston is in the second position, the motor being controlled by a telemetric signal received from a remote device.

2. The implantable, motorized device of Claim 1 wherein the integrated reservoir is selected from a group consisting of a balloon, an elastic polymer, a flexible bladder, a rubber container, a silicone container, and combinations thereof.

3. The implantable, motorized device of Claim 1 or 2 further comprising an external reservoir, coupled to the fluid port, for filling the integrated reservoir with additional fluid.

4. The implantable, motorized device of Claim 3 wherein the external reservoir is selected from a group consisting of a balloon, an elastic polymer, a flexible bladder, a rubber container, a silicone container, a non-elastomeric container, a bellows, and combinations thereof.

5. The implantable, motorized device of any one of the preceding Claims wherein the motor comprises a non-magnetic electrical drive or a magnetic motor.

6. The implantable, motorized device of any one of the preceding Claims further comprising a first flow control device coupled to the fluid port for controlling movement of the fluid when filling the gastric band,
preferably wherein the first flow control device is selected from a group consisting of a valve, a tube, and a regulator, and combinations thereof.

7. The implantable, motorized device of Claim 6 depending from Claim 3 or 4 further comprising a second flow control device coupled to the external reservoir for controlling movement of the fluid when filling the gastric band.

8. The implantable, motorized device of any one of the preceding Claims wherein the fluid is selected from a group consisting of a drug, a saline solution, and combinations thereof.

9. The implantable, motorized device of any one of the preceding Claims wherein the motor moves the first leadscrew by rotational movement,
or wherein the first leadscrew comprises a rod and the motor moves the rod by translational movement.

10. The implantable, motorized device of any one of the preceding Claims further comprising a seal connected to the first piston and positioned between the first piston and the integrated reservoir, and/or
further comprising a slidable seal connected to the first piston, wherein the slidable seal maintains a seal with the housing as the first piston moves from the first position to the second position.

11. The implantable, motorized device of any one of the preceding Claims wherein the first piston is positioned between the first leadscrew and the integrated reservoir.

12. The implantable, motorized device of any one of the preceding Claims further comprising a second leadscrew coupled to the motor.

13. The implantable, motorized device of any one of the preceding Claims further comprising a second piston coupled to the first leadscrew.

14. The implantable, motorized device of any one of the preceding Claims wherein the housing is made of a material selected from a group consisting of a rigid material and a flexible material, and/or
wherein the housing comprises a plurality of housing walls defining boundaries of the integrated reservoir, and/or
wherein the housing defines a chamber for housing the integrated reservoir, the first piston, the first leadscrew, and the motor.

15. The implantable, motorized device of any one of the preceding Claims wherein the piston does not include a seal.

## Amended claims

### Amended claims in accordance with Rule 137(2) EPC.

1. An implantable, motorized device (440, 540) that facilitates movement of fluid to an inflatable portion of a gastric band (205), the implantable, motorized device comprising:
a housing (450, 550, 850, 950) having a fluid port (454, 554, 854, 954);
an integrated reservoir (452, 558, 852, 952) positioned within the housing (450, 550, 850, 950) and adjacent to the fluid port (454, 554, 854, 954) and capable of holding the fluid;
a first piston (442, 542, 842, 942) positioned within the housing (450, 550, 850, 950) and adjacent to the integrated reservoir (452, 558, 852, 952), the first piston (442, 542, 842, 942) capable of moving a portion of the fluid from the integrated reservoir (452, 558, 852, 952) to the inflatable portion of the gastric band (205); and
a first leadscrew (446, 546, 846, 946) coupled to the first piston (442, 542, 842, 942) and causing the first piston (442, 542, 842, 942) to move between a first position and a second position;
**characterized by** further comprising
a motor (444, 544, 844, 944) attached to the housing (450, 550, 850, 950) and coupled to the first leadscrew (446, 546, 846, 946), the motor moving the first piston (442, 542, 842, 942) between the first position and the second position, and when the first piston (442, 542, 842, 942) is in the first position, the integrated reservoir (452, 558, 852, 952) having a larger volume than when the first piston (442, 542, 842, 942) is in the second position, the motor (444, 544, 844, 944) being controlled by a telemetric signal received from a remote device.

2. The implantable, motorized device (440, 540) of Claim 1 wherein the integrated reservoir (452, 558, 842, 942) is selected from a group consisting of a balloon, an elastic polymer, a flexible bladder, a rubber container, a silicone container, and combinations thereof.

3. The implantable, motorized device of Claim 1 or 2 further comprising an external reservoir (964), coupled to the fluid port (454, 554, 854, 954), for filling the integrated reservoir (452, 552, 858, 952) with additional fluid.

4. The implantable, motorized device of Claim 3 wherein the external reservoir (964) is selected from a group consisting of a balloon, an elastic polymer, a flexible bladder, a rubber container, a silicone container, a non-elastomeric container, a bellows, and combinations thereof.

5. The implantable, motorized device (440, 540) of any one of the preceding Claims wherein the motor (444, 544, 844, 944) comprises a non-magnetic electrical drive or a magnetic motor.

6. The implantable, motorized device of any one of the preceding Claims further comprising a first flow control (861) device coupled to the fluid port for controlling movement of the fluid when filling the gastric band,
preferably wherein the first flow control device (861) is selected from a group consisting of a valve, a tube, and a regulator, and combinations thereof.

7. The implantable, motorized device of Claim 6 depending from Claim 3 or 4 further comprising a second flow control device (960) coupled to the external reservoir (964) for controlling movement of the fluid when filling the gastric band.

8. The implantable, motorized device (440, 540) of any one of the preceding Claims wherein the fluid is selected from a group consisting of a drug, a saline solution, and combinations thereof.

9. The implantable, motorized device (440, 540) of any one of the preceding Claims wherein the motor (444, 544, 844, 944) moves the first leadscrew (446, 546, 846, 946) by rotational movement,
or wherein the first leadscrew (446, 546, 846, 946) comprises a rod and the motor moves the rod by translational movement.

10. The implantable, motorized device (440, 540) of any one of the preceding Claims further comprising a seal connected to the first piston (442, 542, 842, 942) and positioned between the first piston (442, 542, 842, 942) and the integrated reservoir (452, 558, 852, 952), and/or
further comprising a slidable seal connected to the first piston (442, 542, 842, 942), wherein the slidable seal maintains a seal with the housing (450, 550, 850, 950) as the first piston (442, 542, 842, 942) moves from the first position to the second position.

11. The implantable, motorized device (440, 540) of any one of the preceding Claims wherein the first piston (442, 542, 842, 942) is positioned between the first leadscrew (446, 546, 846, 946) and the integrated reservoir (452, 558, 852, 952).

12. The implantable, motorized device (440, 540) of any one of the preceding Claims further comprising a second leadscrew coupled to the motor (444, 544, 844, 944).

13. The implantable, motorized device (440, 540) of any one of the preceding Claims further comprising a second piston coupled to the first leadscrew (446, 546, 846, 946).

14. The implantable, motorized device (440, 540) of any one of the preceding Claims wherein the housing (450, 550, 850, 950) is made of a material selected from a group consisting of a rigid material and a flexible material, and/or
wherein the housing (450, 550, 850, 950) comprises a plurality of housing walls defining boundaries of the integrated reservoir (452, 558, 852, 952), and/or
wherein the housing (450, 550, 850, 950) defines a chamber for housing the integrated reservoir (452, 558, 852, 952), the first piston (442, 542, 842, 942), the first leadscrew (446, 546, 846, 946), and the motor (444, 544, 844, 944).

15. The implantable, motorized device (440, 540) of any one of the preceding Claims wherein the piston (442, 542, 842, 942) does not include a seal.
